# FASCICULE DE BREVET EUROPEEN

(11) **EP 1 661 550 B1**
(45) Date de publication et mention de la délivrance du brevet: **27.04.2011**
(21) Numéro de dépôt: 05022402.1
(22) Date de dépôt: 13.10.2005
(51) Int. Cl.: A61K 8/42, A61K 8/81, A61Q 19/00

(54) **Composition cosmétique et/ou dermatologique contenant un composé d'urée**
Kosmetische und/oder dermatologische Zusammensetzung enthaltend eine Harnstoffverbindung
Cosmetic and/or dermatological composition comprising urea compound

(30) Priorité: 09.11.2004 FR 0452575
(43) Date de publication de la demande: 31.05.2006
(73) Titulaire: L'OREAL, S.A., 75008 Paris (FR)
(72) Inventeur: Fonolla Moreno, Angeles, 75013 Paris (DE)
(74) Mandataire: Duvert, Sandra

(56) Documents cités:
- EP-A- 1 535 607
- WO-A-01/89591
- WO-A1-2006/018198
- DE-A1- 2 703 185
- DE-A1-102004 039 550
- FR-A- 2 795 083
- US-A1- 2004 064 026

## Description

L'invention se rapporte à une composition pour application topique, notamment cosmétique et/ou dermatologique, contenant dans un milieu aqueux, un composé d'urée hydroxylé et un polymère réticulé comportant au moins un monomère à groupement sulfonique.

L'invention se rapporte aussi à un procédé de traitement de la peau d'être humain, comprenant l'application d'une composition cosmétique selon l'invention sur les matières kératiniques, en particulier la peau et/ou les muqueuses.

On sait que la peau a tendance à se dessécher du fait de facteurs environnementaux (pollution, vent, froid, air conditionné), psychologiques (fatigue, stress) ou hormonaux (ménopause). Or, il est important que la peau soit bien hydratée et ne subisse pas de perte en eau risquant d'entraîner un flétrissement et un dessèchement de la peau. Aussi, il est courant d'incorporer dans les compositions cosmétiques, des substances qui provoquent une réhydratation de la peau par captation de l'eau atmosphérique et par rétention de l'eau dans la peau.

Une telle amélioration de l'hydratation peut être réalisée de plusieurs manières, et en particulier soit à l'aide d'actifs apportant de l'eau, tels que les polyols et notamment la glycérine, soit à l'aide d'actifs protégeant le film hydrolipidique de la peau et créant ainsi un effet barrière, ce qui évite la perte en eau de la peau.

La glycérine est un bon agent hydratant ; elle présente toutefois l'inconvénient d'induire aux compositions la contenant, un effet collant qui peut être rédhibitoire si la quantité de glycérine est trop importante. Pour pallier cet inconvénient, il est connu d'associer à la glycérine, d'autres agents hydratants tels que les dérivés d'urée. Des dérivés d'urée particulièrement intéressants sont les composés d'urée hydroxylés qui n'ont pas l'effet collant de la glycérine.

Ces actifs sont incorporés dans les supports classiques des compositions pour application topique. Les compositions classiquement utilisées dans les domaines cosmétique et/ou dermatologique sont notamment des émulsions eau-dans-huile (E/H), des émulsions huile-dans-eau (H/E), ou des gels aqueux. Pour stabiliser et/ou épaissir ces compositions, il est connu d'y ajouter des polymères gélifiants. Or, il a été observé par la demanderesse que les composés d'urée hydroxylés présentent l'inconvénient de déstabiliser les compositions contenant les polymères habituellement utilisés dans le domaine cosmétique ou dermatologique, tels que les polymères carboxyvinyliques du type carbomer (nom CTFA) ou les polymères carboxyvinyliques comportant un groupement hydrophobe, tels que les produits commercialisés sous les dénominations Pemulen ou Carbopol 1342 (nom CTFA: Acrylates/C10-30 akyl acrylate crosspolymer) par la société Novéon.

Il subsiste donc le besoin de disposer de compositions cosmétiques et/ou dermatologiques gélifiées, qui soient stables en présence de composés d'urée hydroxylés.

La demanderesse a découvert de manière surprenante que les compositions contenant un composé d'urée hydroxylé pouvaient être stabilisées et/ou épaissies avec un polymère à fonction sulfonique sans que ne se produisent une déstabilisation et/ou une perte de viscosité.

Ainsi, l'invention a pour objet une composition cosmétique et/ou dermatologique contenant dans un milieu aqueux, (a) au moins un composé de formule (I) suivante : 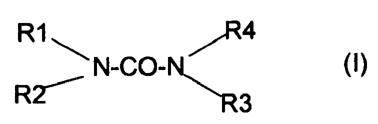 dans laquelle :
R1, R2, R3 et R4 représentent chacun, indépendamment l'un de l'autre, un atome d'hydrogène, un groupe alkyle en Cl-C4 ou un groupe hydroxyalkyle en C2-C6 pouvant contenir de 1 à 5 groupes hydroxyles, où au moins un des radicaux R1 à R4 représente un groupe hydroxyalkyle,
ainsi que leurs sels, leurs solvats, et leurs isomères,
et (b) au moins un polymère réticulé comportant au moins un monomère à groupement sulfonique qui est l'acide 2-acrylamido 2-méthylpropane sulfonique.

La composition de l'invention présente l'avantage de n'être pas collante, tout en étant stable, c'est-à-dire qu'il ne se produit ni séparation de phase ni décantation ni perte de la viscosité.

On entend ici par « milieu aqueux », un milieu contenant de l'eau et éventuellement un ou plusieurs solvants organiques. En outre, la composition de l'invention étant une composition cosmétique et/ou dermatologique, ce milieu est un milieu physiologiquement acceptable. On entend ici par « milieu physiologiquement acceptable », un milieu compatible avec la peau, les muqueuses, les yeux. Il s'agit de préférence d'un milieu cosmétiquement acceptable, c'est-à-dire qui, en outre, présente une couleur, une odeur et un toucher agréables et qui ne génère pas d'inconforts inacceptables (picotements, tiraillements, rougeurs), susceptibles de détourner la consommatrice d'utiliser cette composition.

Par ailleurs, le milieu aqueux de cette composition contient un pourcentage d'eau d'au moins 30 % en poids, par exemple de 30 à 99,8 % en poids, de préférence de 30 à 90 % en poids, mieux de 30 à 70 % en poids et encore mieux de 30 à 60 % en poids par rapport au poids total de la composition.
De façon avantageuse, le pH du milieu aqueux est compatible avec les matières kératiniques et notamment avec la peau. Ce pH va de préférence de 3 à 8,5 et mieux de 3,5 à 7,5, préférentiellement de 5 à 7, et plus préférentiellement allant de 5 à 6.
Par ailleurs, on entend par « polymère » dans la présente demande, aussi bien un homopolymère qu'un copolymère. Comme indiqué ci-après, il peut s'agir d'un polymère libre ou partiellement ou entièrement neutralisé.

Le polymère utilisé dans la composition selon l'invention permet d'obtenir une composition gélifiée stable et ayant en outre de bonnes propriétés cosmétiques.

Aussi, l'invention a encore pour objet l'utilisation d'un polymère réticulé comportant au moins un monomère à groupement sulfonique qui est l'acide 2-acrylamido 2-méthylpropane sulfonique, pour gélifier une composition cosmétique et/ou dermatologique contenant un composé de formule (I) suivante : 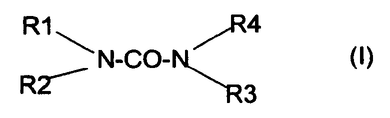 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle,
ainsi que leurs sels, leurs solvats, et leurs isomères.

### Composé d'urée hydroxylé

Le composé d'urée présent dans la composition selon l'invention est un composé de formule (I) suivante : 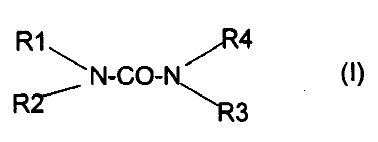 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle,
ainsi que leurs sels, leurs solvats, et leurs isomères.

Pour les composés de formule (I) :
- De préférence R1 désigne un groupe hydroxyalkyle en C2-C6 et R2, R3, R4 désignent, indépendamment l'un de l'autre, un atome d'hydrogène ou un groupe alkyle en C1-C4 ;
- Préférentiellement R1 désigne un groupe hydroxyalkyle en C2-C6 comprenant de 1 à 5 groupes hydroxyles, notamment 1 groupe hydroxyle, et R2, R3, R4 désignent un atome d'hydrogène ;
- Plus préférentiellement, R1 désigne un groupe hydroxyalkyle en C2-C4 comprenant 1 groupe hydroxyle et R2, R3, R4 désignent un atome d'hydrogène.

Comme groupes alkyle en C1-C4, on peut citer notamment les groupes méthyle, éthyle, n-propyle, isopropyle, n-butyle, iso-butyle et tert-butyle.

Parmi les groupes hydroxyalkyle en C1-C6, on préfère ceux contenant un seul groupe hydroxyle et en particulier les groupes hydroxyéthyle, hydroxypropyle, hydroxybutyle, hydroxypentyle et hydroxyhexyle.

Comme sels de tels composés, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, acide sulfonique ou acide phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Comme composés de formule (I) préférés, on peut citer le N-(2-hydroxyéthyl)-urée ; le N-(2-hydroxypropyl)-urée ; le N-(3-hydroxypropyl)-urée ; le N-(2,3-dihydroxypropyl)-urée ; le N-(2,3,4,5,6-pentahydroxyhexyl)-urée; le N-méthyl-N-(1,3,4,5,6-pentahydroxy-2-hexyl)-urée ; le N-méthyl-N'-(1-hydroxy-2-méthyl-2-propyl)-urée; le N-(1-hydroxy-2-méthyl-2-propyl)-urée ; le N-(1,3-dihydroxy-2-propyl)-urée ; le N-(tris-hydroxyméthyl-méthyl)-urée ; le N-éthyl-N'-(2-hydroxyéthyl)-urée ; le N,N-bis-(2-hydroxyéthyl)-urée ; le N,N'-bis-(2-hydroxyéthyl)-urée ; le N,N-bis-(2-hydroxypropyl)-urée; le N,N'-Bis-(2-hydroxypropyl)-urée ; le N,N-Bis-(2-hydroxyéthyl)-N'-propyl-urée ; le N,N-Bis-(2-hydroxypropyl)-N'-(2-hydroxyéthyl)-urée ; le N-tert.Butyl-N'-(2-(hydroxyéthyl)-N'-(2-(hydroxypropyl)-urée; le N-(1,3-dihydroxy-2-propyl)-N'-(2-hydroxyéthyl)-urée ; le N,N-Bis-(2-hydroxyéthyl)-N',N'-dimethyl-urée; le N,N,N'.N'-tetrakis-(2-hydroxyéthyi)-urée ; le N',N'-Bis-(2-hydroxyéthylrN',N'-bis-(2-hydroxypropyl)-urée ; et leurs mélanges.

De préférence, le composé de formule (I) est le N-(2-hydroxyéthyl)-urée.

Les composés de formule (I) sont des composés connus et notamment décrits dans la demande DE-A-2703185. Parmi ceux-ci, le N-(2-hydroxyéthyl)-urée est en outre disponible dans le commerce, sous forme de mélange à 50% en poids dans l'eau, auprès de la société National Starch sous la dénomination commerciale Hydrovance^{®}.

Le composé de formule (I) peut être présent dans la composition selon l'invention en une teneur allant de 0,1 % à 50 % en poids, par rapport au poids total de la composition, de préférence allant de 0,1 % à 20 % en poids, et préférentiellement allant de 0,1 % à 10 % en poids.

### Polymère réticulé comportant au moins un monomère à groupement sulfonique

Les polymères comportant au moins un monomère à groupement sulfonique, utilisés dans la composition de l'invention sont hydrosolubles ou hydrodispersibles ou gonflables dans l'eau. Les polymères utilisés conformément à l'invention peuvent être des homopolymères ou des copolymères et sont susceptibles d'être obtenus à partir d'au moins un monomère à insaturation éthylénique et à groupement sulfonique, pouvant être sous forme libre ou partiellement ou totalement neutralisée. Ces polymères peuvent éventuellement comprendre au moins un groupement hydrophobe et constituer alors un polymère amphiphile (ou polymère modifié hydrophobe).

De façon préférentielle, les polymères conformes à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés », des polymères totalement ou pratiquement totalement neutralisés, c'est-à-dire neutralisés à au moins 90 %.

Les polymères utilisés dans la composition de l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Le monomère à groupement sulfonique du polymère utilisé dans la composition de l'invention est l'acide 2-acrylamido 2-méthylpropane sulfonique ainsi que ses formes partiellement ou totalement neutralisées, et leurs mélanges.
Les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Lorsque les polymères utilisés sont des homopolymères, ils ne comportent que des monomères à groupement sulfonique et un ou plusieurs agents de réticulation.

Ces homopolymères sont généralement neutralisés, et ils peuvent être obtenus selon le procédé de préparation comprenant les étapes suivantes :
(a) on disperse ou on dissout le monomère tel que l'acide 2-acrylamido 2-méthylpropane sulfonique sous forme libre dans une solution de tertio-butanol ou d'eau et de tertio-butanol ;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Les homopolymères d'AMPS préférés sont en général caractérisés par le fait qu'ils comprennent, distribués de façon aléatoire :
a) de 90 à 99,9% en poids de motifs de formule générale (II) suivante : 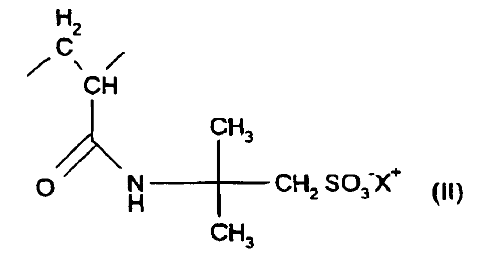 dans laquelle X⁺ désigne un proton, un cation de métal alcalin, un cation alcalino-terreux ou l'ion ammonium, au plus 10% mol des cations X⁺ pouvant être des protons H⁺;
b) de 0,01 à 10% en poids de motifs réticulants provenant d'au moins un monomère ayant au moins deux double-liaison oléfiniques ; les proportions en poids étant définis par rapport au poids total du polymère.

Les homopolymères selon l'invention plus particulièrement préférés comprennent de 98 à 99,5 % en poids de motifs de formule (II) et de 0,2 à 2 % en poids de motifs réticulants.

Comme polymères de ce type, on peut citer notamment l'homopolymère réticulé et neutralisé d'acide 2-acrylamido 2-méthylpropane sulfonique, commercialisé par la société Clariant sous la dénomination commerciale « Hostacerin AMPS » (nom CTFA : ammonium polyacryldimethyltauramide).

Le polymère peut être aussi un homopolymère amphiphile (ou homopolymère modifié hydrophobe) choisi parmi les polymères amphiphiles statistiques d'AMPS modifiés par réaction avec une n-monoalkylamine ou une di-n-alkylamine en C₆₋C₂₂, tels que ceux décrits dans le document WO-A-00/31154, qui sont des homopolymères greffés.

Lorsque les polymères utilisés sont des copolymères, ils sont susceptibles d'être obtenus à partir de des monomères à insaturation éthylénique et à groupement sulfonique et d'autres monomères à insaturation éthylénique, c'est-à-dire de monomères à insaturation éthylénique sans groupement sulfonique.

Les monomères à insaturation éthylénique et à groupement sulfonique sont choisis parmi ceux décrits ci-dessus.

Les monomères à insaturation éthylénique sans groupement sulfonique peuvent être choisis parmi les monomères hydrophiles à insaturation éthylénique, les monomères hydrophobes à insaturation éthylénique et leurs mélanges. Quand le polymère contient des monomères hydrophobes, il constitue un polymère amphiphile (appelé aussi polymère modifié hydrophobe).

Les monomères hydrophiles à insaturation éthylénique peuvent être choisis par exemple parmi les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, le vinylformamide, l'anhydride maléique, l'acide itaconique, l'acide maléique ou les mélanges de ces composés.

Quand le polymère de la composition selon l'invention est un copolymère susceptible d'être obtenu à partir de monomères à insaturation éthylénique et à groupement sulfonique et de monomères hydrophiles à insaturation éthylénique, il peut être choisi notamment parmi (1) les copolymères anioniques réticulés d'acrylamide ou méthylacrylamide et d'acide 2-acrylamido-2-méthylpropane sulfonique, notamment ceux se présentant sous la forme d'une émulsion E/H, tels que ceux commercialisés sous le nom de SEPIGEL 305 par la société Seppic (nom C.T.F.A. : Polyacrylamide / C13-14 Isoparaffin / Laureth-7), sous le nom de SIMULGEL 600 par la société Seppic (nom C.T.F.A. : Acrylamide / Sodium acryloyldimethyltaurate copolymer / Isohexadecane / Polysorbate 80), (2) les copolymères d'acide(méth)acrylique ou de (méth)acrylate et d'acide 2-acrylamido-2-méthylpropane sulfonique, notamment ceux se présentant sous la forme d'une émulsion E/H et (3) les copolymères d'acide 2-acrylamido-2-méthylpropane sulfonique et de vinylpyrrolidone ou de vinylformamide, tels que les produits commercialisés sous les dénominations ARISTOFLEX AVC par la société Clariant.

Quand les monomères à groupement sulfonique sont copolymérisés avec des monomères hydrophobes à insaturation éthylénique comportant une chaîne hydrophobe, appelée aussi chaîne grasse (chaîne en C6-C50), le polymère obtenu est amphiphile, c'est-à-dire qu'il comporte à la fois une partie hydrophile et une partie hydrophobe. On appelle aussi de tels polymères, des polymères modifiés hydrophobes.

Ces polymères modifiés hydrophobes peuvent contenir en outre un ou plusieurs monomères ne comportant ni groupement sulfonique ni chaîne grasse, tels que les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, le vinylformamide, l'anhydride maléique, l'acide itaconique, l'acide maléique ou les mélanges de ces composés.

Comme polymères modifiés hydrophobes, on peut utiliser notamment ceux susceptibles d'être obtenus à partir d'acide 2-acrylamido-2-méthylpropane sulfonique (AMPS) et d'au moins un monomère hydrophobe à insaturation éthylénique comportant au moins un groupement ayant de 6 à 50 atomes de carbone, plus préférentiellement de 6 à 22 atomes de carbone, encore plus préférentiellement de 6 à 18 atomes de carbone et plus particulièrement 12 à 18 atomes de carbone.

Ces polymères sont décrits notamment dans les documents EP-A-750899, US-A-5089578 et WO-A-2002/:43689, et dans les publications de Yotaro Morishima suivantes :
- « Self-assembling amphiphilic polyelectrolytes and their nanostructures - Chinese Journal of Polymer Science Vol. 18, N°40, (2000), 323-336 »;
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol-33, N° 10 - 3694-3704 » ;
- « Solution properties of miscelle networks formed by non-ionic moieties cavalently bound to an polyelectrylyte: salt effects on rheological behavior - Langmuir, 2000, Vol.16, N°12, 5324-5332 »;« Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Les monomères hydrophobes de ces polymères particuliers sont choisis de préférence parmi les acrylates, alkylacrylates, acrylamides ou alkylacrylamides de formule (III) suivante : 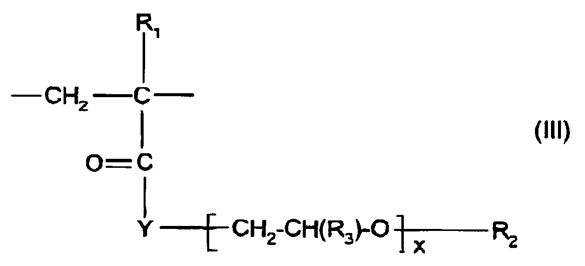 dans laquelle R₁ et R₃, identiques ou différents, désignent un atome d'hydrogène ou un radical alkyle substantiellement linéaire ou ramifié en C₁-C₆ (de préférence méthyle) ; Y désigne O ou NH ; R₂ désigne un radical hydrocarboné comportant de 6 à 50 atomes de carbone, plus préférentiellement de 6 à 22 atomes de carbone, encore plus préférentiellement de 6 à 18 atomes de carbone et plus particulièrement de 12 à 18 atomes de carbone ; x désigne un nombre de moles d'oxyde d'alkylène et varie de 0 à 100.

Le radical R₂ est choisi de préférence parmi les radicaux alkyles en C₆-C₁₈ substantiellement linéaires (par exemple n-hexyle, n-octyle, n-décyle, n-hexadécyle, n-dodécyle ou lauryle, n-octadécyle ou stéaryle), ramifiés ou cycliques (par exemple cyclododécane (C₁₂) ou adamantane (C₁₀)) : les radicaux alkylperfluorés en C₆-C₁₈ (par exemple le groupement de formule -(CH₂)₂-(CF₂)₉₋CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles substantiellement linéaires et plus particulièrement le radical n-dodécyle, n-hexadecyle ou n-octadécyle, et leurs mélanges.

Selon une forme particulièrement préférée de l'invention, le monomère de formule (III) comporte au moins un motif oxyde d'alkylène (x ≥ 1) et de préférence plusieurs motifs oxyde d'alkylène (x > 1) constituant une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés (ou nombre de moles d'oxyde d'alkylène) varie en général de 3 à 100, plus préférentiellement de 3 à 50 et encore plus préférentiellement de 7 à 25.

Parmi ces polymères, on peut citer :
- les copolymères réticulésneutralisés ou non, comportant de 15 à 60 % en poids de motifs AMPS et de 40 à 85 % en poids de motifs (C₈-C₁₆)alkyl(méth)acrylamide ou de motifs (C₈-C₁₆)alkyl(méth)acrylate par rapport au polymère, tels que ceux décrits dans le document EP-A-750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, par rapport au polymère, tels que ceux décrits dans le document US-A-5,089,578.
- les copolymères réticulés d'AMPS partiellement ou totalement neutralisé et de n-dodécylméthacrylamide, tels que ceux décrits dans les articles de Morishima cités ci-dessus.

Comme polymères modifiés hydrophobes, on peut citer plus particulièrement les copolymères constitués (i) de motifs acide 2-acrylamido-2-méthylpropane-sulfonique (AMPS) de formule (II) indiquée ci-dessus, dans laquelle X⁺ est un proton, un cation de métal alcalin, un cation alcalino-terreux ou l'ion ammonium, et (ii) de motifs de formule (IV) suivante : 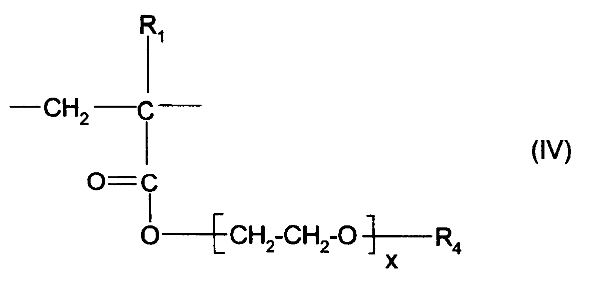 dans laquelle x désigne un nombre entier variant de 3 à 100, de préférence de 3 à 50 et plus préférentiellement de 7 à 25 ; R₁ a la même signification que celle indiquée ci-dessus dans la formule (III) et R₄ désigne un radical alkyle linéaire ou ramifié comportant de 6 à 22 atomes de carbone et de préférence de 10 à 22 atomes de carbone.

Les polymères modifiés hydrophobes de ce type sont notamment ceux décrits dans les articles de Morishima mentionnés ci-dessus, pour lesquels x = 25, R₁ désigne méthyle et R₄ représente n-dodécyle ; ou ceux décrits dans le document

WO-A-02/43689, pour lesquels x = 8 ou 25, R₁ désigne méthyle et R₄ représente n-hexadécyle (C₁₆), n-octadécyle (C₁₈), ou n-dodécyle (C₁₂), ou leurs mélanges. Les polymères pour lesquels X+ désigne le sodium ou l'ammonium sont plus particulièrement préférés.

Les polymères modifiés hydrophobes préférés pouvant être utilisés dans la composition conforme à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH = 2,2-Azo-Bis-[2-Amidinopropane] Hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ces polymères modifiés hydrophobes peuvent être notamment obtenus par polymérisation radicalaire en milieu tert-butanol dans lequel ils précipitent, En utilisant la polymérisation par précipitation dans le tert-butanol, il est possible d'obtenir une distribution de la taille des particules du polymère particulièrement favorable pour ses utilisations.

La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Ces polymères modifiés hydrophobes préférés sont en particulier ceux décrits dans le document EP-1,069,142, et notamment ceux obtenus par polymérisation de l'acide 2-acrylamido-2-mëthylpropane-sulfonique (AMPS) ou l'un de ses sels de sodium ou d'ammonium, avec un ester de l'acide (méth)acrylique et
- d'un alcool en C₁₀-C₁₈ oxyéthyléné par 8 moles d'oxyde d'éthylène (GENAPOL® C-080 de la société CLARIANT),
- d'un alcool oxo en C₁₁ oxyéthyléné par 8 moles d'oxyde d'éthylène (GENAPOL® UD-080 de la société CLARIANT),
- d'un alcool oxo en C₁₁ oxyéthyléné par 7 moles d'oxyde d'éthylène (GENAPOL® UD-070 de la société CLARIANT),
- d'un alcool en C₁₂-C₁₄ oxyéthyléné par 7 moles d'oxyde d'éthylène (GENAPOL® LA-070 de la société CLARIANT),
- d'un alcool en C₁₂-C₁₄ oxyéthyléné par 9 moles d'oxyde d'éthylène (GENAPOL® LA-090 de la société CLARIANT),
- d'un alcool en C₁₂-C₁₄ oxyéthyléné par 11 moles d'oxyde d'éthylène (GENAPOL® LA-110 de la société CLARIANT),
- d'un alcool en C₁₆-C₁₈ oxyéthyléné par 8 moles d'oxyde d'éthylène (GENAPOL® T-080 de la société CLARIANT),
- d'un alcool en C₁₆-C₁₈ oxyéthyléné par 15 moles d'oxyde d'éthylène (GENAPOL® T-150 de la société CLARIANT),
- d'un alcool en C₁₆-C₁₈ oxyéthyléné par 11 moles d'oxyde d'éthylène (GENAPOL® T-110 de la société CLARIANT),
- d'un alcool en C₁₆-C₁₈ oxyéthyléné par 20 moles d'oxyde d'éthylène (GENAPOL® T-200 de la société CLARIANT),
- d'un alcool en C₁₆-C₁₈ oxyéthyléné par 25 moles d'oxyde d'éthylène (GENAPOL® T-250 de la société CLARIANT),
- d'un alcool en C₁₈-C₂₂ oxyéthyléné par 25 moles d'oxyde d'éthylène et/ou d'un alcool iso-C₁₆-C₁₈ oxyéthyléné par 25 moles d'oxyde d'éthylène.

La concentration molaire en % des motifs de formule (II) et des motifs de formule (IV) dans les polymères selon l'invention varie en fonction de l'application cosmétique souhaitée et des propriétés rhéologiques de la formulation recherchées. Elle peut varier entre 0,1 et 99,9 % en moles.

De préférence pour les polymères les plus hydrophobes, la proportion molaire en motifs de formule (II) ou (IV) varie de 50,1 à 99,9 %, plus particulièrement de 70 à 95 % et encore plus particulièrement de 80 à 90 %.

De préférence pour les polymères peu hydrophobes, la proportion molaire en motifs de formule (II) ou (IV) varie de 0,1 à 50 %, plus particulièrement de 5 à 25 % et encore plus particulièrement de 10 à 20 %.

La distribution des monomères dans les polymères de l'invention peut être, par exemple, alternée, bloc (y compris multibloc) ou quelconque.

Comme polymères modifiés hydrophobes de ce type, on peut citer notamment le copolymère d'AMPS et de méthacrylate de stéaryle éthoxylé (25 EO) (copolymère réticulé par le trimethylolpropane triacrylate, obtenu à partir de Genapol T-250 et d'AMPS) (nom CTFA: Ammonium Acryloyldimethyltaurate / Steareth-25 Methacrylate Crosspolymer) commercialisé sous la dénomination ARISTOFLEX HMS par la société Clariant.

Les polymères comportant au moins un monomère à groupement sulfonique, utilisés dans la composition conforme à l'invention, sont présents dans des quantités en matière active allant par exemple de 0,01 à 20 % en poids, de préférence de 0,1 à 10 % en poids, préférentiellement de 0,1 à 5 % en poids et plus préférentiellement encore de 0,5 à 2 % en poids par rapport au poids total de la composition.

### Additifs

En plus de l'eau, le milieu de la composition de l'invention peut contenir un ou plusieurs solvants organiques qui peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des alcools monohydriques linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer les dérivés de polypropylène glycol (PPG) tels que les esters de polypropylône glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Selon le but recherché, les compositions selon l'invention peuvent ne contenir qu'un milieu aqueux (eau, solvants et additifs hydrophiles) ou bien elles peuvent contenir aussi des constituants lipophiles tels que les corps gras comme les huiles, et les additifs lipophiles.

Ainsi, les compositions selon l'invention peuvent se présenter sous toutes les formes appropriées pour une application topique, notamment sous forme de gels aqueux, sous forme d'émulsions obtenues par dispersion d'une phase grasse (appelée aussi phase huileuse) dans une phase aqueuse (H/E) ou inversement (E/H) ou d'émulsions multiples (par exemple E/H/E ou H/E/H ou H/H/E). Elles peuvent être plus ou moins fluides et avoir l'aspect d'une crème blanche ou colorée, d'une pommade, d'un lait, d'une lotion, d'un sérum, d'une pâte. Ces compositions sont préparées selon les méthodes usuelles.

Selon un mode particulier de réalisation de l'invention, la composition selon l'invention se présente sous forme d'une émulsion et comporte alors au moins une phase huileuse. La proportion de phase aqueuse peut aller de 20 à 99 %, de préférence de 50 à 98 % et mieux de 60 à 98 % en poids par rapport au poids total de la composition.

La proportion de la phase huileuse de l'émulsion peut aller de 1 à 80 % en poids, de préférence de 2 à 50 % en poids et mieux de 2 à 40 % en poids par rapport au poids total de la composition. Les corps gras de la phase huileuse, notamment les huiles, et les émulsionnants et co-émulsionnants éventuellement présents, utilisés dans la composition sous forme d'émulsion sont choisis parmi ceux classiquement utilisés dans le domaine cosmétique ou dermatologique. L'émulsionnant et le co-émulsionnant, quand ils sont présents, le sont généralement, en une proportion allant de 0,1 à 30 % en poids, de préférence de 0,3 à 20 % en poids et mieux de 0,5 à 15 % en poids par rapport au poids total de la composition. L'émulsion peut en outre, contenir des vésicules lipidiques en plus ou à la place des émulsionnants et/ou co-émulsionnants.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H. on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Coming, et comme les alkyl-dimethicone copolyols tels que le Laurylmethicone copolyol vendu sous la dénomination "Dow Coming 5200 Formulation Aid" par la société Dow Coming, le Cetyl dimethicone copolyol vendu sous la dénomination Abil EM 90^{R} par la société Goldschmidt et le mélange de Polyglyceryl-4 isostearate/Cetyl dimethicone copolyol/Hexyl laurate vendu sous la dénomination Abil WE 09^{R} par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; les esters d'acides gras et de sorbitan oxyalkylénés comprenant par exemple de 20 à 100 OE, et par exemple ceux commercialisés sous les dénominations commerciales Tween 20 ou Tween 60 par la société Uniqema ; les éthers d'alcools gras oxyalkylénés (oxyéthylénés et/ou oxypropylénés) ; les esters de sucres, alcoxylés ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces émulsionnants comme par exemple le mélange de stéarate de glycéryle et de stéarate de PEG-100 (nom CTFA : Glyceryl Stearate / PEG-100 Stearate) commercialisé sous la dénomination Arlacel 165 par la société Uniqema et sous la dénomination SIMULSOL 165 par la société SEPPIC ; ou le mélange de tartrate de dimyristyle, d'alcool cétéarylique, de Pareth-7 et de PEG-25 laureth-25, commercialisé sous la dénomination Cosmacol PSE par la société Sasol (nom CTFA: Dimyristyl tartrate / cetearyl alcool /12-15 Pareth 7 / PPG 25 laureth 25).

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition, en utilisant des composés appropriés, par exemple les polymères amphiphiles indiqués ci-dessus comme polymères utilisables selon l'invention.

Quand la composition de l'invention est sous forme d'émulsion, elle comporte au moins une phase huileuse qui contient au moins une huile, notamment une huile cosmétique. On entend par "huile" un corps gras liquide à la température ambiante (25°C).

Comme huiles utilisables dans la composition de l'invention, on peut utiliser par exemple les huiles hydrocarbonées d'origine animale, telles que le perhydrosqualène (ou squalane) ; les huiles hydrocarbonées d'origine végétale, telles que les triglycérides des acides caprylique/caprique comme ceux vendus par la société Stearineries Dubois ou ceux vendus sous les dénominations Miglyol 810, 812 et 818 par la société Dynamit Nobel ou encore les huiles d'origine végétale, par exemple les huiles de tournesol, de maïs, de soja, de courge, de pépins de raisin, de sésame, de noisette, d'abricot, de macadamia, d'arara, de coriandre, de ricin, d'avocat, l'huile de jojoba, l'huile de beurre de karité; les huiles de synthèse ; les huiles de silicone comme les polyméthylsiloxanes (PDMS) volatiles ou non à chaîne siliconée linéaire ou cyclique, liquides ou pâteux à température ambiante; les huiles fluorées telles que celles partiellement hydrocarbonées et/ou siliconées comme celles décrites dans le document JP-A-2-295912 ; les éthers tels que l'éther dicaprylique (nom CTFA : Dicaprylyl ether) ; et les benzoates d'alcools gras en C₁₂-C₁₅ (Finsolv TN de FINETEX) ; leurs mélanges.

La phase huileuse peut aussi comporter un ou plusieurs corps gras choisi par exemple parmi les alcools gras (alcool cétylique, l'alcool stéarylique, alcool cétéarylique), les acides gras (acide stéarique) ou les cires (paraffine, cires de polyéthylène, camauba, cire d'abeilles).

La composition selon l'invention peut comprendre tout additif habituellement utilisé dans les compositions cosmétiques ou dermatologiques, tels que par exemple les conservateurs, les charges, les agents pour ajuster le pH (bases ou acides), les filtres solaires. Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Comme charges qui peuvent être utilisées dans la composition de l'invention, on peut citer par exemple, les pigments ; la poudre de silice; le talc; les particules de polyamide et notamment celles vendues sous la dénomination ORGASOL par la société Atochem ; les poudres de polyéthylène ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch : les microsphères à base de copolymères acryliques, telles que celles en copolymère diméthacrylate d'éthylène glycol/methacrylate de lauryle vendues par la société Dow Coming sous la dénomination de POLYTRAP ; les poudres de polymethylmethacrylate telles que celles coomercialisées sous la dénomination MICROPEARL M 100 par la société Matsumoto; les poudres expansées telles que les microsphères creuses et notamment, les microsphères commercialisées sous la dénomination EXPANCEL par la société Kemanord Piast ou sous la dénomination MICROPEARL F 80 ED par la société Matsumoto ; les microbilles de résine de silicone telles que celles commercialisées sous la dénomination TOSPEARL par la société Toshiba Silicone ; les poudres de polyuréthanne telles que la poudre de copolymère hexaméthylène diisocyanate/trimethylol hexyllactone commercialisée sous la dénomination Plastic Powder D-400 par la société Toshiba Pigment (Nom CTFA : HDI / Trimethylol Hexyllactone Crosspolymer) ; et leurs mélanges. Quand elles sont présentes, ces charges peuvent être en des quantités allant de 0,001 à 20 % en poids, de préférence de 0,1 à 10 % en poids et mieux de 1 à 5 % en poids par rapport au poids total de la composition.

La composition selon l'invention peut constituer un produit pour le soin de la peau, notamment pour le visage, le cou, le contour de l'oeil, le corps ; ou bien encore un produit de maquillage de la peau tel qu'un produit du teint (notamment fond de teint), un fard à paupières, un fard à joue, un eye-liner, un produit anticemes, un produit de maquillage du corps : ou encore un produit de protection solaire avec incorporation de filtres ; ou bien un produit de nettoyage de la peau.

La composition est généralement non rincée, mais elle peut être rincée si elle constitue un produit de nettoyage notamment moussant.

L'invention a aussi pour objet un procédé de traitement cosmétique d'une matière kératinique telle que la peau, des cils, des sourcils, des ongles ou des muqueuses, caractérisé en ce qu'on applique sur la matière kératinique, une composition telle que définie ci-dessus.

L'invention a encore pour objet l'utilisation de la composition telle que définie ci-dessus, pour préparer une pommade ou un onguent destiné à traiter thérapeutiquement le visage et/ou le corps humain.

### Packaging :

Selon un autre aspect, l'invention concerne également un ensemble cosmétique comprenant :
- i): un récipient délimitant au moins un compartiment, ledit récipient étant fermé par un élément de fermeture ; et
- ii): une composition telle que décrite précédemment et disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur, notamment sous forme d'une brosse comportant un arrangement de poils maintenus par un fil torsadé. Une telle brosse torsadée est décrite notamment dans le brevet US-4,887,622. Il peut être également sous forme d'un peigne comportant une pluralité d'éléments d'application, obtenus notamment de moulage. De tels peignes sont décrits par exemple dans le brevet FR-2,796,529. L'applicateur peut être sous forme d'un pinceau, tel que décrit par exemple dans le brevet FR-2,722,380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US-5,492,426. L'applicateur peut être solidaire du récipient, tel que décrit par exemple le brevet FR-2,761,959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO-A-01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène. Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO-A-031018423 ou dans le brevet FR-2,791,042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR-2,792,618.

L'invention a aussi pour objet, un ensemble cosmétique ou dermatologique comprenant :
a) un récipient délimitant au moins un compartiment, ledit récipient étant fermé par un élément de fermeture; et
b) une composition disposée à l'intérieur dudit compartiment, la composition étant telle que définie ci-dessus.

L'invention est illustrée plus en détails par les exemples non limitatifs décrits ci-après. Les quantités sont indiquées en pourcentage en poids.

| Composition (Emulsion H/E) | Exemple 1 selon l'invention | Exemple 2 selon l'invention | Exemple 3 comparatif | Exemple 4 comparatif |
|---|---|---|---|---|
| *PHASE A* | | | | |
| Eau | Qsp 100% | Qsp 100 % | Qsp 100 % | Qsp 100 % |
| Hostacerin AMPS | 1 % | 1% | - | - |
| Carbopol 980 | - | - | 0,5% | 0,5% |
| Conservateur | 0,3 % | 0,3 % | 0,3 % | 0,3 % |
| | | | | |

| *PHASE B* | | | | |
|---|---|---|---|---|
| Cyclohexasiloxane | 10 % | 10 % | 10 % | 10 % |
| Caprylic/capric triglycérides | 5 % | 5% | 5% | 5 % |
| Beurre de Karité | 1 % | 1 % | 1 % | 1 % |
| PEG-20 methylglucose sesquistearate | 2,5 % | 2,5 % | 2,5 % | 2,5 % |
| Conservateur | 0,25% | 0.25% | 0.25% | 0.25% |
| | | | | |

| *PHASE C* | | | | |
|---|---|---|---|---|
| Triéthanolamine | - | - | 0,25% | 0,25 % |
| | | | | |

| *PHASE D* | | | | |
|---|---|---|---|---|
| Polymethylmethacrylate (charge) | 2 % | 2 % | 2 % | 2 % |
| | | | | |

| PHASE E | | | | |
|---|---|---|---|---|
| N-(2-hydroxyéthyl)-urée | 7% | 14% | 7% | 14 % |
| Viscosité (mobile 3) | 21 poises | 24 poises | 17 poises | 14 poises |
| mesurée 24 heures après la préparation | (2,1 Pa.s) | (2,4 Pa.s) | (1,7 Pa.s) | (1,4 Pa.s) |

Mode opératoire : on chauffe séparément les composants de la phase A et de la phase B. On verse la phase A dans la phase B et on mélange jusqu'à la formation de l'émulsion. Pour le gel au carbopol (exemples 3 et 4), on neutralise avec la phase C. On refroidit jusqu'à 50 °C et on ajoute la phase D, en dispersant bien la charge. On ajoute ensuite la phase E et on continue d'agiter jusqu'à homogénéisation totale de la composition.

Ce tableau montre que les émulsions H/E selon l'invention ont une viscosité satisfaisante qui ne varie pas avec l'augmentation du taux de N-(2-hydroxyéthyl)-urée, tandis que les exemples comparatifs qui contiennent du carbopol 980, polymère carboxyvinylique, ont une viscosité plus faible, qui, en outre, chute de façon linéaire avec l'augmentation du taux de N-(2-hydroxyéthyl)-urée.

## Revendications

1. Composition cosmétique et/ou dermatologique contenant dans un milieu aqueux, (a) au moins un composé de formule (I) suivante : 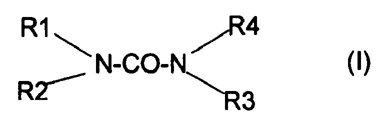 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle,
ainsi que leurs sels, leurs solvats, et leurs isomères,
et (b) au moins un polymère réticulé comportant au moins un monomère à groupement sulfonique qui est l'acide 2-acrylamido 2-méthylpropane sulfonique.

2. Composition selon la revendication 1, **caractérisée par le fait que** pour les composés de formule (I), R1 désigne un groupe hydroxyalkyle en C2-C6 et R2, R3, R4 désignent, indépendamment l'un de l'autre, un atome d'hydrogène ou un groupe alkyle en C1-C4.

3. Composition selon la revendication 1 ou 2, **caractérisée en ce que** pour les composés de formule (I), R1 désigne un groupe hydroxyalkyle en C2-C6 comprenant de 1 à 5 groupes hydroxyles, et R2, R3, R4 désignent un atome d'hydrogène.

4. Composition selon la revendication précédente, **caractérisée en ce que** R1 désigne un groupe hydroxyalkyle en C2-C6 comprenant 1 groupe hydroxyle.

5. Composition selon l'une quelconque des revendications précédentes, **caractérisée en ce que** pour les composés de formule (I), R1 désigne un groupe hydroxyalkyle en C2-C4 comprenant 1 groupe hydroxyle et R2, R3, R4 désignent un atome d'hydrogène.

6. Composition selon l'une quelconque des revendications précédentes, **caractérisée en ce que** le composé de formule (I) est choisi parmi le N-(2-hydroxyéthyl)-urée ; le N-(2-hydroxypropyl)-urée ; le N-(3-hydroxypropyl)-urée ; le N-(2,3-dihydroxypropyl)-urée ; le N-(2,3,4,5,6-pentahydroxyhexyl)-urée ; le N-méthyl-N-(1,3,4,5,6-pentahydroxy-2-hexyl)-urée ; le N-méthyl-N'-(1-hydroxy-2-méthyl-2-propyl)-urée ; le N-(1-hydroxy-2-méthyl-2-propyl)-urée; le N-(1,3-dihydroxy-2-propyl)-urée ; le N-(tris-hydroxyméthyl-méthyl)-urée ; le N-éthyl-N'-(2-hydroxyéthyl)-urée ; le N,N-bis-(2-hydroxyéthyl)-urée ; le N,N'-bis-(2-hydroxyéthyl)-urée ; le N,N-bis-(2-hydroxypropyl)-urée ; le N,N'-Bis-(2-hydroxypropyl)-urée; le N,N-Bis-(2-hydroxyéthyl)-N'-propyl-urée ; le N,N-Bis-(2-hydroxypropyl)-N'-(2-hydroxyéthyl)-urée ; le N-tert.Butyl-N'-(2-(hydroxyéthyl)-N'-(2-(hydroxypropyl)-urée; le N-(1,3-dihydroxy-2-propyl)-N'-(2-hydroxyéthyl)-urée; le N,N-Bis-(2-hydroxyéthyl)-N',N'-dimethyl-urée ; le N,N,N',N'-tetrakis-(2-hydroxy-éthyl)-urée ; le N',N'-Bis-(2-hydroxyéthyl)-N',N'-bis-(2-hydroxypropyl)-urée : et leurs mélanges.

7. Composition selon l'une quelconque des revendications précédentes, **caractérisée en ce que** le composé de formule (I) est le N-(2-hydroxyéthyl)-urée.

8. Composition selon l'une quelconque des revendications précédentes, **caractérisée en ce que** les sels des composés de formule (I) sont choisis parmi les sels de l'acide sulfurique, l'acide chlorhydrique, l'acide bromhydrique, l'acide iodhydrique, l'acide phosphorique, l'acide borique, l'acide propionique, l'acide acétique, l'acide téréphtalique, l'acide citrique, l'acide tartrique.

9. Composition selon l'une quelconque des revendications précédentes, **caractérisée en ce que** le composé de formule (I) est présent en une teneur allant de 0,1 à 50 % en poids par rapport au poids total de la composition.

10. Composition selon l'une quelconque des revendications précédentes, **caractérisée en ce que** le polymère est un homopolymère réticulé et neutralisé d'acide 2-acrylamido 2-méthylpropane sulfonique.

11. Composition selon l'une quelconque des revendications précédentes, **caractérisée en ce que** le polymère comportant au moins un monomère à groupement sulfonique est un homopolymère amphiphile choisi parmi les polymères amphiphiles statistiques d'acide 2-acrylamido 2-méthylpropane sulfonique modifiés par réaction avec une n-monoalkylamine ou une di-n-alkylamine en C₆-C₂₂.

12. Composition selon l'une quelconque des revendications précédentes, **caractérisée en ce que** le polymère comportant au moins un monomère à groupement sulfonique est choisi parmi les copolymères anioniques réticulés d'acrylamide ou méthylacrylamide et d'acide 2-acrylamido-2-méthylpropane sulfonique, les copolymères d'acide(méth)acrylique ou de (méth)acrylate et d'acide 2-acrylamido-2-méthylpropane sulfonique, et les copolymères d'acide 2-acrylamido-2-méthylpropane sulfonique et de vinylpyrrolidone ou de vinylformamide.

13. Composition selon l'une quelconque des revendications précédentes, **caractérisée en ce que** le polymère comportant au moins un monomère à groupement sulfonique est un polymère modifié hydrophobe susceptible d'être obtenu à partir d'acide 2-acrylamido 2-méthylpropane sulfonique et d'au moins un monomère hydrophobe à insaturation éthylénique comportant au moins un groupement ayant de 6 à 50 atomes de carbone, de préférence de 6 à 22 atomes de carbone.

14. Composition selon la revendication précédente, **caractérisée en ce que** le monomère hydrophobe à insaturation éthylénique est choisi parmi les acrylates ou les acrylamides de formule (III) suivante : 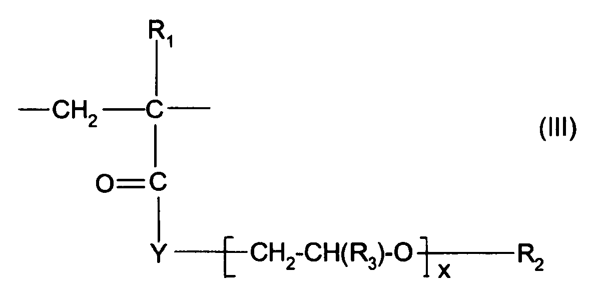 dans laquelle R₁ et R₃, identiques ou différents, désignent un atome d'hydrogène ou un radical alkyle substantiellement linéaire ou ramifié en C₁-C₆ ; Y désigne O ou NH ; R₂ désigne un radical hydrocarboné comportant de 6 à 50 atomes de carbone ; x désigne un nombre de moles d'oxyde d'alkylène et varie de 0 à 100.

15. Composition selon la revendication précédente, **caractérisée en ce que** le radical hydrocarboné R₂ est choisi parmi les radicaux alkyles en C₆-C₁₈, substantiellement linéaires, ramifiés ou cycliques ; les radicaux alkylperfluorés en C₆-C₁₈; le radical cholestéryle ou un ester de cholestérol ; les groupes polycycliques aromatiques.

16. Composition selon la revendication 14 ou 15, **caractérisée en ce que** pour le monomère de formule (III) comporte au moins un motif oxyde d'alkylène (x ≥ 1).

17. Composition selon l'une quelconque des revendications 13 à 16, **caractérisée en ce que** le polymère modifié hydrophobe est choisi parmi :
- les copolymères réticulés neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(meth)acrylate, par rapport au polymère ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, par rapport au polymère ;
- les copolymères réticulés d'AMPS partiellement ou totalement neutralisé et de n-dodécylméthacrylamide.

18. Composition selon la revendication 13, **caractérisée en ce que** le polymère modifié hydrophobe est choisi parmi les copolymères constitués de motifs acide 2-acrylamido-2-méthylpropane-sulfonique de formule (II) : 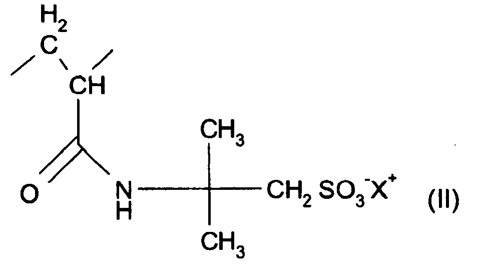 dans laquelle X⁺ est un proton, un cation de métal alcalin, un cation alcalino-terreux ou l'ion ammonium, et
(ii) de motifs de formule (IV) suivante : 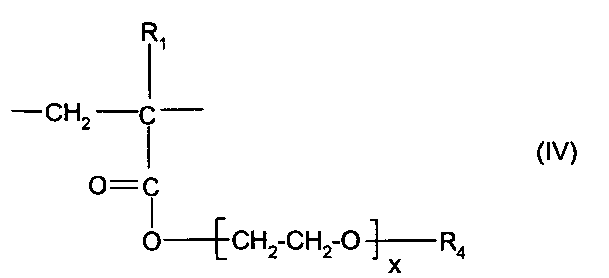 dans laquelle x désigne un nombre entier variant de 3 à 100, de préférence de 5 à 80 et plus préférentiellement de 7 à 25 ; R₁ a la même signification que celle indiquée ci-dessus dans la formule (III) et R₄ désigne un radical alkyle linéaire ou ramifié comportant de 6 à 22 atomes de carbone et de préférence de 10 à 22 atomes de carbone.

19. Composition selon l'une quelconque des revendications précédentes, **caractérisée en ce que** la quantité de polymère comportant au moins un monomère à groupement sulfonique va de 0,01 à 20 % en poids, de préférence de 0,1 à 10 % en poids par rapport au poids total de la composition.

20. Composition selon l'une quelconque des revendications précédentes, **caractérisée en ce qu'**elle comprend au moins 30 % d'eau en poids par rapport au poids total de la composition.

21. Composition selon l'une quelconque des revendications précédentes, **caractérisée en ce qu'**elle comprend en plus au moins une phase huileuse.

22. Composition selon l'une quelconque des revendications précédentes, **caractérisée en ce qu'**elle constitue un produit pour le soin de la peau, un produit de maquillage de la peau, un produit de protection solaire, un produit de nettoyage de la peau.

23. Procédé de traitement cosmétique d'une matière kératinique, **caractérisé par le fait qu'**on applique sur la matière kératinique, une composition cosmétique selon l'une quelconque des revendications 1 à 22.

24. Utilisation de la composition selon l'une quelconque des revendications 1 à 22, pour préparer une pommade ou un onguent destiné à traiter thérapeutiquement le visage et/ou le corps humain.

25. Ensemble cosmétique ou dermatologique comprenant :
a) un récipient délimitant au moins un compartiment, ledit récipient étant fermé par un élément de fermeture ; et
b) une composition disposée à l'intérieur dudit compartiment, la composition étant conforme à l'une quelconque des revendications 1 à 22.

26. Utilisation d'un polymère comportant au moins un monomère à groupement sulfonique qui est l'acide 2-acrylamido 2-méthylpropane sulfonique , pour gélifier une composition cosmétique et/ou dermatologique contenant un composé de formule (I) suivante : 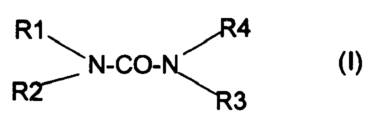 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle,
ainsi que leurs sels, leurs solvats, et leurs isomères.

## Claims

1. Cosmetic and/or dermatological composition containing, in an medium, (a) art least one compound of formula (I) below: 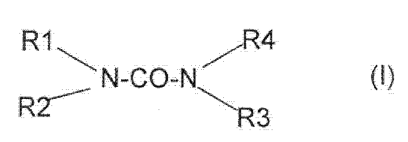 in which:
R1, R2, R3 and R4 represent, of each other, a hydrogen atom, a C1-C4 alkyl group or a C2-C6 hydroxyalkyl group possible containing from 1 to 5 hydroxyl groups, in which at least one of the radicals R1 to R4 represents a hydroxyalkyl group,
and also the salts, solvates and thereof,
and (b) at least one crosslinked polymer comprising at least one monomer containing a sulfonic group which is 2-acrylamido-2-methylpropanesulfonic acid.

2. according to Claim 1, **characterized in that**, for the compounds of formula (I), R1 denotes a C2-C6 hydroxyalkyl group and R2, R3 and R4 denote, independently of each other, a hydrogen or a C1-C4 alkyl group.

3. Composition according to Claim 1 or 2, **characterized in that**, for the compounds of formula (I) , R1 denotes a C2-C6 hydroxyalkyl group comprising from 1 to 5 hydroxyl groups and R2, R3 and R4 denote a hydrogen atom.

4. Composition according to the preceding claim, **characterized in that** R1 denotes a C2-C6 b-ydrcxyalkyl group comprising hydroxyl group.

5. Compositions according to any one of the preceding claims, **characterized in that**, for the compounds of formula (I), R1 denotes a C2-C4 hydroxyalkyl group comprising one hydroxy group and R2, R3 and R4 denote a hydrogen atom.

6. compositions according to any one of the preceding claims, **characterized in that** the compound of formula (I) is chosen from N-(2-hydroxyethyl) urea; N-(2-hydroxypropyl) urea; N-(3-hydroxypropyl) urea; N-(2,3-dihydroxypropyl) urea ; N- 2,3,4,5,6-pentahydroxyhexyl urea; N-methyl-N-(1,3,4,5,6-pentahydroxy-2-hexyl)urea; N-methyl-N'-(1-hydroxy-2-methyl-2-propyl)urea; N-(1-hydroxy-2-methyl-2-propyl)urea; N-(1,3-dihydroxy-2-propyl)urea; N-[tris(hydroxymethyl)methyl]urea; N-ethyl-N'-(2-hydroxyethyl)urea; N,N-bis 2-hydroxyethyl)urea; N,N'-bis (2-hydroxyethyl)urea; N,N-bis(2-hydroxypropyl)urea; N,N'-bis(2-hydroxypropyl)urea; N,N-bis(2-hydroxyethyl)-N'-propylurea; N,N-bis(2-hydroxypropyl)-N'-(2-hydroxyethyl)urea; N-tert-butyl-N'-(2-hydroxyethyl-)-N'-(2-hydroxypropyl)urea; N-(1,3-dihydroxy-2-propyl)-N'-(2-hydroxyethyl)urea; N,N-bis(2-hydroxyethyl)-N',N'-dimethylurea; N,N,N'N'-tetrakis(2-hydroxyethyl)urea; N'N'-bis(2-hydroxyethyl)-N',N'-bis(2-hydroxypropyl)urea; and thereof.

7. Composition according to any one of the preceding claims, **characterized in that** the of formula (I) is N-(2-hydroxyethyl)urea.

8. Composition according to any one of the preceding claims, **characterized in that** the salts of the compounds of formula (I) are chosen from the salts of sulfuric acid, hydrochloric acid, acid, hydriodic acid, acid, boric acid, propionic acid, ascetic acid, acid, citric acid tartaric acid.

9. Composition according to any one of the preceding claims, **characterized in that** the compound of formula (I) is present in a content from 0.1% to 50% by weight relative to the total of composition.

10. Composition according to any one of the preceding claims, **characterized in that** the polymer is a crosslinked and neutralized 2-acrylamido-2-methylpropanesulfonic acid homopolymer.

11. Composition according to any one of the preceding claims, **characterized in that** the polymer comprising at least one containing a sulfonic group is an amphiphilic homopolymer chosen from random amphiphilic polymers of 2-acrylamido-2-methylpropanesulfonic acid modifies by with a C₆-C₂₂ mono-n-alkylamine or din-alkylamine.

12. compositions according to any one of the preceding claims, **characterized in that** the polymer comprising at least one containing a sulfonic group is chosen from crosslinked anionic copolymers of acrylamide oar methacrylamide and of 2-acrylamido-2-methylpropanesulfonic acid, copolymers of (meth) acrylic acid or of (meth)acrylate and of 2-acrylamido-2-methylpropanesulfonic acid, and copolymers of 2-acrylamido-2-methylpropanesulfonic acid and of vinylpyrrolidone or of vinylformamide.

13. Composition according to any one of the preceding claims, **characterized in that** the polymer comprising at least one containing a sulfonic is a hydrophobic modified polymer that may be obtained from 2-acrylamido-2-methylpropanesulfonic acid and from at least one ethylenically unsaturated hydrophobic monomer comprising at least one containing from 6 to 50 carbon atoms and from 6 to 22 carbon atoms.

14. Composition according to the preceding claim, **characterized in that** the ethylenically unsaturated hydrophobic monomer is chosen from the acrylates or acrylamides of formula (III) below: 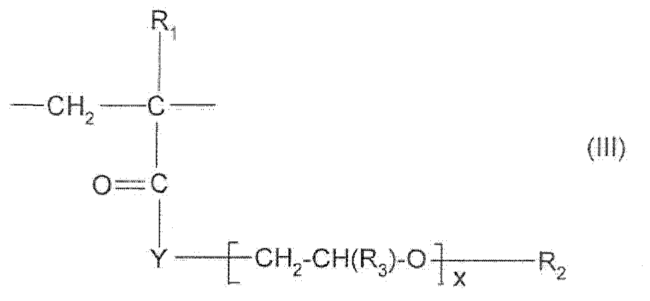 in which R₁ and R₃, which may be identical or different, denote a hydrogen atom or a substantially linear or branched C₁-C₆ alkyl radical; Y denotes O or NH; R₂ denotes a hydrocarbon-based radical containing from 6 to 50 carbon atoms; x denotes a number of moles of alkylene oxide and ranges from 0 to 100.

15. Composition according to the preceding claim, **characterized in that** the hydrocarbon-based radical R₂ is chosen from substantially linear, branched or cyclic C₆-C₁₈ alkyl radicals; C₆-C₁₈ perfluoroalkyl radicals; the cholesteryl radical or a cholesterol ester; aromatic polycyclic groups.

16. Composition according to claim 14 or 15, **characterized in that** the monomer of formula (III) comprises at least one alkylene oxide unit (x ≥ 1).

17. Composition according to any one of Claims 13 to 16, **characterized in that** the hydrophobic modified polymer is chosen from:
- crosslinked, neutralized or noun-neutralized copolymers comprising from 15% to 60% by weight of AMPS units and from 40% to 85% by weight of (C₈-C₁₆)alkyl(meth)acrylamide units or of (C₈-C₁₆)alkyl (meth)acrylate units, relative to the polymer;
- terpolymers comprising from 10 mol% to 90 mol% of acrylamide units, from 0.1 mol% to 10 mol% of AMPS units and from 5 mol% to 80 mol% of n-(C₆-C₁₈)alkylacrylamide units, relative to the polymer;
- crosslinked copolymer of partially or totally neutralized AMPS and of n-dodecylmethacrylamide.

18. Composition according to claim 13, **characterized in that** the hydrophobic modified polymer is chosen copolymers consisting of 2-acrylamido-2-methylpropanesalfonic acid units of formula (II): 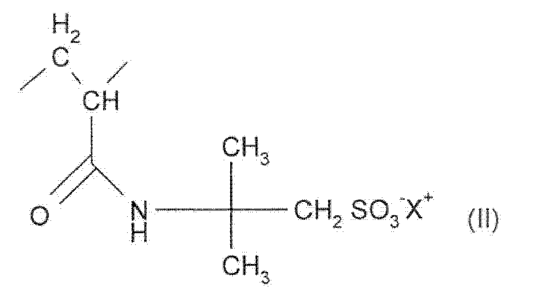 in which X⁺ is a proton, an alkali metal cation, an alkaline-earth metal cation or an ammonium ion, and
(ii) of units of formula (IV) bellow: 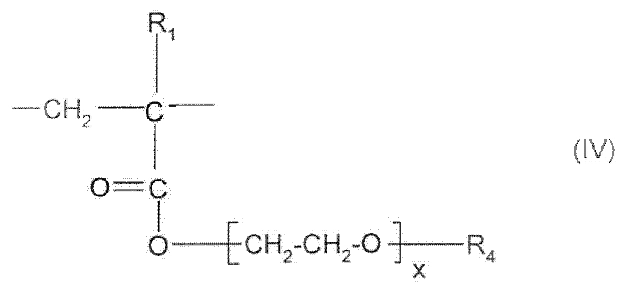 in which x denotes an integer ranging from 3 to 100, preferably from 5 to 80 and more from 7 to 25; R₁ has the same meaning as that indicated above in (III) and R₄ denotes a linear or branched alkyl radical containing from 6 to 22 carbon atoms and preferably from 10 to 22 atoms.

19. Composition according to any one of the claims, **characterized in that** the amount of polymer comprising at least one monomer containing a sulfonic group ranges from 0.01% to 20% by weight and preferable from 0.1% to 10% by weight relative to the total weight of the composition.

20. Composition according to any one of the preceding claims, **characterized in that** it comprises at least 30% by weight of water relative to the total weight of composition.

21. Composition according to any one of the preceding claims, **characterized in that** it also comprises at least one oily phase.

22. Composition according to any one of the preceding claims, **characterized in that** it is a skincare product, a skin makeup produce, an antisum product or a skin product.

23. Cosmetic process for treating a keratin material, **characterized in that** a cosmetic according to any one of Claims 1 to 22 is applies to the keratin maternal.

24. use of the composition according to any one of claims 1 to 22, for preparing a pomade or an ointment for the therapeutic treatment of the human face and/or body.

25. Cosmetic or dermatological comprising:
a) a container delimiting at least one compartment, the said container being closed by of a closing member; and
b) a composition plated inside the said compartment, the composition being in accordance with any one of Claims 1 to 22.

26. Use of a polymer comprising at least one monomer containing a sulfonic group which is 2-acrylamido-2-methylpropanesulfonic acid, to gel a cosmetic and/or dermatological composition containing a compound of formula (I) below: 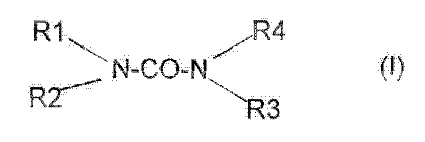 in which:
R1, R2, R3 and R4 represent, independently of each other, a hydrogen atom, a C1-C4 alkyl group or a C2-C6 hydroxyalkyl group possibly containing from 1 to 5 hydroxyl groups, in which at least one of the radicals R1 to R4 represents a hydroxyalkyl group,
and also the salts, solvates and isomers thereof.

## Patentansprüche

1. Kosmetische und/oder dermatologische Zusammensetzung, die in einem wässrigen Medium enthält:
(a) mindestens eine Verbindung der folgenden Formel (I): 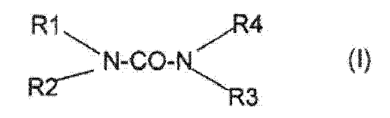 in der Formel:
R1, R2, R3 et R4 bedeuten jeweils unabhängig voneinander ein Wasserstoffatom, eine C₁₋₄-Alkylgruppe oder eine C₂₋₆-Hydroxyalkylgruppe, die 1 bis 5 Hydroxygruppen enthalten kann, wobei mindestens eine der Gruppen R1 bis R4 eine Hydroxyalkylgruppe bedeutet,
sowie ihre Salze, ihre Solvate und ihre Isomere,
und (b) mindestens ein vernetztes Polymer, das mindestens ein Monomer mit Sulfonsäuregruppe enthält, bei dem es sich um die 2-Acrylamido-2-methylpropansulfonsäure handelt.

2. Zusammensetzung nach Anspruch 1, **dadurch gekennzeichnet, dass** für die Verbindungen der Formel (I) R1 eine C₂₋₆-Hydroxyalkylgruppe bedeutet und R2, R3, R4 unabhängig voneinander ein Wasserstoffatom oder eine C₁₋₄-Alkylgruppe bedeuten.

3. Zusammensetzung nach Anspruch 1 oder 2, **dadurch gekennzeichnet, dass** für die Verbindungen der Formel (I) R1 eine C₂₋₆-Hydroxyallkylgruppe bedeutet, die 1 bis 5 Hydroxygruppen enthält, und R2, R3, R4 ein Wasserstoffatom bedeuten.

4. Zusammensetzung nach dem vorhergehenden Anspruch, **dadurch gekennzeichnet, dass** R1 eine C₂₋₆-Hydroxyalkylgruppe die 1 Hydroxygruppe enthält.

5. Zusammensetzung nach einem der vorhergehenden Ansprüche, **dadurch gekennzeichnet, dass** für die Verbindungen der Formel (I) R1 eine C₂₋₄-Hydroxyalkylgruppe bedeutet, die 1 Hydroxygruppe enthält, und R2, R3, R4 ein Wasserstoffatom bedeuten.

6. Zusammensetzung nach einem der vorhergehenden Ansprüche, **dadurch gekennzeichnet, dass** die Verbindung der Formel (I) ausgewählt ist unter: N-(2-Hydroxyethyl)-harnstoff; N-(2-Hydroxypropyl)-harnstoff; N-(3-Hydroxypropyl)-harnstoff; N-(2,3-Dihydroxypropyl)-harnstoff; N-(2,3,4,5,6-Pentahydroxyhexyl)-harnstoff; N-Methyl-N-(1,3,4,5,6-pentahydroxy-2-hexyl)-harnstoff; N-Methyl-N'-(1-hydroxy-2-methyl-2-propyl)-harnstoff; N-(1-Hydroxy-2-methyl-2-propyl)-harnstoff: N-(1,3-Dihydroxy-2-propyl)-harnstoff; N-(Tris-hydroxymethyl-methyl)-harnstoff; N-Ethyl-N'-(2-hydroxyethyl)-harnstoff; N,N-Bis(2-hydroxyethyl)-harnstoff; N,N'-Bis(2-hydroxyethyl)-harnstoff; N,N-Bis(2-hydroxypropyl)-harnstoff N,N'-Bis(2-hydroxypropyl)-harnstoff; N,N-Bis(2-hydroxethyl)-N'-propyl-harnstoff; N,N-Bis(2-hydroxypropyl)-N'-(2-hydroxyethyl)-harnstoff; N-tert-Butyl-N'-(2-(hydroxyethyl)-N'-(2-(hydroxypropyl)-harnstoff; N-(1,3-Dihydroxy-2-propyl)-N'-(2-hydroxyethyl)-harnstoff; N,N-Bis(2-hydroxyethyl)-N',N'-dimethyl-harnstoff; N,N,N',N'-Tetrakis-(2-hydroxy-ethyl)-harnstoff; N',N'-Bis(2-hydroxyethyl)-N',N'-bis-(2-hydroxypropyl)-harnstoff; und ihren Gemischen.

7. Zusammensetzung nach einem der vorhergehenden Ansprüche, **dadurch gekennzeichnet, dass** die Verbindung der Formel (I) der N-(2-Hydroxyethyl)-harnstoff ist.

8. Zusammensetzung nach einem der vorhergehenden Ansprüche, **dadurch gekennzeichnet, dass** die Salze der Verbindungen der Formel (I) unter den Salzen der Schwefelsäure, Salzsäure, Bromwasserstoffsäure, Iodwasserstoffsäure, Phosphorsäure, Borsäure, Propionsäure, Essigsäure, Terephthalsäure, Citronensäure oder Weinsäure ausgewählt sind.

9. Zusammensetzung nach einem der vorhergehenden Ansprüche, **dadurch gekennzeichnet, dass** die Verbindung der Formel (I) in einem Mengenanteil von 0,1 bis 50 Gew. -%, bezogen auf das Gesamtgewicht der Zusammensetzung, enthalten ist.

10. Zusammensetzung nach einem der vorhergehenden Ansprüche, **dadurch gekennzeichnet, dass** das Polymer ein vernetztes und neutralisiertes Homopolymer der 2-Acrylamido-2-methylpropan-sulfonsäure ist,

11. Zusammensetzung nach einem der vorhergehenden Ansprüche, **dadurch gekennzeichnet, dass** das Polymer, das mindestens ein Monomer mit Sulfonsäuregruppe enthält, ein amphiphiles Homopolymer ist, das unter den statistischen amphiphilen Polymeren der 2-Acrylamido-2-methylpropansulfonsäure ausgewählt ist, die durch Reaktion mit einem n-Monoalkylamin oder einem Di-n-alkylamin mit 6 bis 22 Kohlenstoffatomen modifiziert sind.

12. Zusammensetzung nach einem der vorhergehenden Ansprüche, **dadurch gekennzeichnet, dass** das Polymer, das mindestens ein Monomer mit Sulfonsäuregruppe enthält, unter den vernetzten anionischen Copolymeren von Acrylamid oder Methacrylamid und der 2-Acrylamido-2-methylpropansulfonsäure, den Copolymeren der (Meth)acrylsäure oder des (Meth)acrylats und der 2-Acrylamido-2-methylpropansulfonsäure und den Copolymeren der 2-Acrylamido-2-methylpropansulfonsäure und Vinylpyrrolidon oder Vinylformamid ausgewählt ist.

13. Zusammensetzung nach einem der vorhergehenden Ansprüche, **dadurch gekennzeichnet, dass** das Polymer, das mindestens ein Monomer mit Sulfonsäuregruppe enthält, ein hydrophob modifiziertes Polymer ist, das ausgehend von 2-Acrylamido-2-methylpropansulfonsäure und mindestens einem hydrophoben Monomer mit ethylenisch ungesättigter Bindung erhältlich ist, das mindestens eine Gruppe mit 6 bis 50 Kohlenstoffatomen und vorzugsweise 6 bis 22 Kohlenstoffatomen aufweist.

14. Zusammensetzung nach dem vorhergehenden Anspruch, **dadurch gekennzeichnet**, das hydrophobe Monomer mit ethylenisch ungesättigter Bindung unter den Acrylaten oder Acrylamiden der folgenden Formel (III) ausgewählt ist: 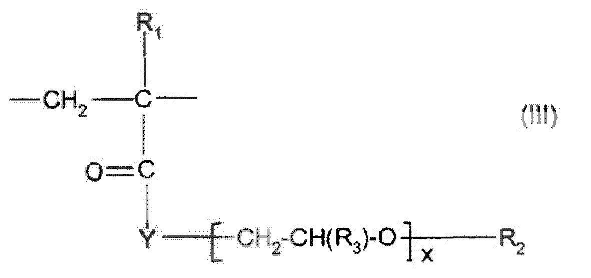 worin R₁ und R₃, die gleich oder verschieden sind, ein Wasserstoffatom oder eine in etwa lineare oder verzweigte Alkylgruppe mit 1 bis 6 Kohlenstoffatomen bedeuten; Y O oder NH bedeutet; R₂ eine Kohlenwasserstoffgruppe mit 6 bis 50 Kohlenstoffatomen ist; und x die Molzahl des Alkylenoxids angibt und im Bereich von 0 bis 100 liegt.

15. Zusammensetzung nach dem vorhergehenden Anspruch, **dadurch gekennzeichnet, dass** die hydrophobe Gruppe R₂ unter den in etwa geradkettigen, verzweigten oder cyclischen C₆₋₁₈-Alkylgruppen; Alkyl-perfluorierten Gruppen mit 6 bis 18 Kohlenstoffatomen; der Cholesterylgruppe oder einem Cholesterinester- und aromatischen polycyclischen Gruppen ausgewählt ist.

16. Zusammensetzung nach Anspruch 14 oder 15, **dadurch gekennzeichnet, dass** das Monomer der Formel (HI) ferner mindestens eine Alkylenoxideinheit (x ≥ 1) aufweist.

17. Zusammensetzung nach einem der Ansprüche 13 bis 16, **dadurch gekennzeichnet, dass** das hydrophob modifizierte Polymer ausgewählt ist unter:
- vernetzten, neutralisierten oder nicht neutralisierten Copolymeren, die 15 bis 60 Gew.-% AMPS-Einheiten und 40 bis 85 Gew.-% (C₈₋₁₆)Alkyl(meth)acrylamid-Einheiten oder (C₈₋₁₆)Alkyl(meth)acrylat-Einheiten, bezogen auf das Polymer, enthalten;
- Terpolymeren, die 10 bis 90 Mol-% Acrylamid, 0,1 bis 10 Mol-% AMPS-Einheiten und 5 bis 80 Mol-% n-(C₆₋₁₈)Alkylacrylamideinheiten, bezogen auf das Polymer, enthalten;
- vernetzten Copolymeren von ganz oder teilweise neutralisierter AMPS und n-Dodecylmethacrylamid.

18. Zusammensetzung nach Anspruch 13, **dadurch gekennzeichnet**, das hydrophob modifizierte Polymer unter den Copolymeren ausgewählt ist, die aus 2-Acrylamido-2-methylpropansulfonsäure-Einheiten der folgenden Formel (II): 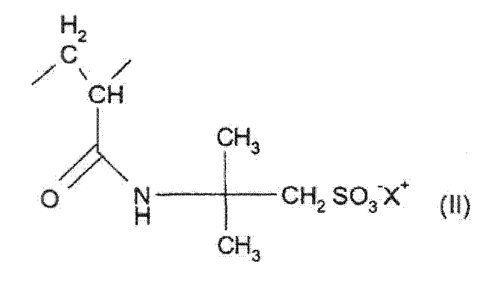 worin X⁺ ein Proton, ein Alkalimetallkation, ein Erdalkalimetallkation oder das Ammoniumion bedeutet, und Einheiten der folgenden Formel (IV) bestehen: 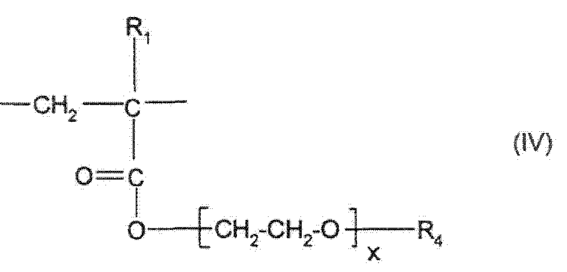 worin x eine ganze Zahl im Bereich von 3 bis 100, vorzugsweise 5 bis 80 und besonders bevorzugt 7 bis 25 ist; R₁ die oben für Formel (III) angegebene Bedeutung aufweist und R₄ eine geradkettige oder verzweigte Alkylgruppe mit 6 bis 22 Kohlenstoffatomen und insbesondere 10 bis 22 Kohlenstoffatomen ist.

19. Zusammensetzung nach einem der vorhergehenden Ansprüche, **dadurch gekennzeichnet, dass** der Mengenanteil des Polymers, das mindestens ein Monomer mit Sulfonsäuregruppe enthält, im Bereich von 0,01 bis 20 Gew.-% und vorzugsweise 0,1 bis 10 Gew.-%, bezogen auf das Gesamtgewicht der Zusammensetzung, liegt.

20. Zusammensetzung nach einem der vorhergehenden Ansprüche, **dadurch gekennzeichnet, dass** sie mindestens 30 Gew.-% Wasser, bezogen auf das Gesamtgewicht der Zusammensetzung, enthält.

21. Zusammensetzung nach einem der vorhergehenden Ansprüche, **dadurch gekennzeichnet, dass** sie ferner mindestens eine Ölphase enthält.

22. Zusammensetzung nach einem der vorhergehenden Ansprüche, **dadurch gekennzeichnet, dass** sie ein Produkt für die Pflege der Haut, ein Produkt zum Schminken der Haut, ein Produkt für den Sonnenschutz, ein Produkt für die Reinigung der Haut ist.

23. Verfahren zur kosmetischen Behandlung einer Keratinsubstanz, **dadurch gekennzeichnet, dass** eine kosmetische Zusammensetzung nach einem der Ansprüche 1 bis 22 auf die Keratinsubstanz aufgetragen wird.

24. Verwendung der Zusammensetzung nach einem der Ansprüche 1 bis 22 für die Herstellung einer Pomade oder Salbe, die für die therapeutische Behandlung des menschlichen Gesichts und/oder Körpers vorgesehen ist.

25. Kosmetische oder dermatologische Einheit, die umfasst:
a. einen Behälter, der mindestens eine Abteilung abgrenzt, wobei der Behälter mit einer Verschlusseinrichtung verschlossen ist, und
b. eine Zusammensetzung, die sich im Inneren der Abteilung befindet, wobei die Zusammensetzung einer Zusammensetzung nach einem der Ansprüche 1 bis 22 entspricht.

26. Verwendung eines Polymers, das mindestens ein Monomer mit Sulfonsäuregruppe enthält, bei dem es sich um die 2-Acrylamido-2-methylpropansulfonsäure handelt, um eine kosmetische und/oder dermatologische Zusammensetzung zu gelieren, die eine Verbindung der folgenden Formel (I) enthält: 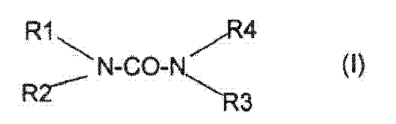 in der Formel:
R1, R2, R3 et R4 bedeuten jeweils unabhängig voneinander ein Wasserstoffatom, eine C₁₋₄-Alkylgruppe oder eine C₂₋₆-Hydroxyalkylgruppe, die 1 bis 5 Hydroxygruppen enthalten kann, wobei mindestens eine der Gruppen R1 bis R4 eine
Hydroxyalkylgruppe bedeutet,
sowie ihre Salze, ihre Solvate und ihre Isomere.
